# EUROPÄISCHE PATENTSCHRIFT

(11) **EP 0 650 520 B1**
(45) Veröffentlichungstag und Bekanntmachung des Hinweises auf die Patenterteilung: **06.11.2002**
(21) Anmeldenummer: 93912629.8
(22) Anmeldetag: 26.06.1993
(51) Int. Cl.: C12N 9/10

(54) **VERFAHREN ZUR HERSTELLUNG VON SACCHAROSE-SYNTHASE**
PROCESS FOR THE PRODUCTION OF SACCHAROSE-SYNTHASE
PROCEDE DE PURIFICATION DE SACCHAROSE-SYNTHASE

(30) Priorität: 01.07.1992 DE 4221595; 16.02.1993 DE 4304558
(43) Veröffentlichungstag der Anmeldung: 03.05.1995
(73) Patentinhaber: Forschungszentrum Jülich GmbH, 52425 Jülich (DE)
(72) Erfinder: ELLING, Lothar, D-52066 Aachen (DE); KULA, Maria-Regina, D-52382 Niederzier (DE)
(86) Internationale Anmeldenummer: DE9300562
(87) Internationale Veröffentlichungsnummer: WO94001540

(56) Entgegenhaltungen:
- JOURNAL OF THE CHINESE BIOCHEMICAL SOCIETY Bd. 17, Nr. 1, 1988, Seiten 42 - 51 RONG-HUAY JUANG ET AL. 'Purification of rice grain sucrose synthetase by preparative electrophoresis' in der Anmeldung erw{hnt
- APPLIED BIOCHEMISTRY AND BIOTECHNOLOGY Bd. 23, Nr. 2, Februar 1990, Seiten 155 - 170 SHARON L. HAYNIE ET AL. 'Enzyme- catalyzed organic synthesis of sucrose and trehalose with in situ regeneration of UDP-glucose' in der Anmeldung erw{hnt
- PLANT PHYSIOLOGY Bd. 45, 1970, Seiten 782 - 786 DEBORAH P. DELMER ET AL. 'The biosynthesis of sucrose and nucleoside diphosphate glucoses in Phaseolus aureus'

## Beschreibung

Saccharose-Synthase (Glycosyltransferase EC 2.4.1.13 UDPG: D-Fructose-2-glucosyltransferase) ist ein insbesondere in Pflanzen (z.B. Weizen, Reis, Mais, Zuckerrüben, etc.) weit verbreitetes, seit langem bekanntes Enzym (siehe z.B. Y. Milner u.a. in "Nature" 206 (1965), S. 825), dessen Funktion als Katalysator zur Bildung aktivierter Zucker innerhalb des Stoffwechsels der Pflanze bereits umfänglich untersucht und zusammenfassend dargestellt worden ist (Avigad, G. in: Loewus, F.A. et al. (eds.) Encyclopedia of Plant Physiology New Series Vol. 13A, Carbohydrates I, Intracellular Carbohydrates, Springer-Verlag, Berlin 1982, 217 - 347). Danach katalysiert das Enzym in vivo die Spaltung von Saccharose gemäß folgender Gleichung 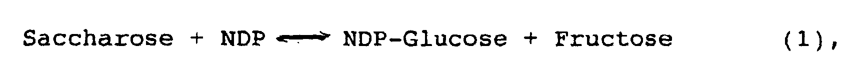 in der N für Nukleoside, wie Uridin, Thymidin, Cytidin, Guanin und Adenin steht.

Reinigung und Eigenschaften des Enzyms wurden u.a. von T. Nomura u.a. in Arch. Biochem. Biophysics 156 (1973) Seiten 644 - 52 beschrieben, die eine Ausbeute von 8,8 % bei 11,4-facher Aufreinigung durch Ammonsulfat-Fällung und Säulenchromatographie an DEAE-Cellulose und Neusilin (MgO • Al₂O₃ • 2SiO₂) erreichen und Kₘ-Werte für Synthese- und Spaltungsreaktion angeben.

Über eine im wesentlichen auf Ammonsulfat-Fällungen basierend gereinigte Saccharose-Synthase und deren Anwendung für die Saccharosesynthese durch Umsetzung und UDP-Glucose und Fructose berichten auch jüngst S.L. Haynie & G.M. Whitesides in Appl. Biochem. & Biotechnol. 23 (1990) Seiten 155 ff. Dabei wird auf die geringe Stabilität des Enzyms (S. 158), insbesondere von hochgereinigten Enzympräparaten hingewiesen (S. 160) sowie auf den Nachteil weit stabilerer Enzym-Präparate von minderer Reinheit auf Grund begleitender Aktivitäten, insbesondere von Phosphoglucomutasen und der infolge niedriger Aktivität bestehenden Notwendigkeit der Anwendung großer Gelvolumina (des gel-immobilisierten Enzyms).

R.H. Juang u.a. beschreiben im J. Chinese Biochem. Soc. 17 (1988) 42 - 51 eine Saccharose-Synthase-Reinigung durch Säulenchromatographie und Elektrophorese mit 38-facher Aufreinigung, die im Hinblick auf die Proteinzusammensetzung durchgeführt wurde.

Trotz der seit langem bekannten Funktionsweise der Saccharose-Synthase und Aufreinigungsverfahren ist die Herstellung aktivierter Zucker gemäß obiger Gleichung (1) wirtschaftlich bislang nicht angewandt worden, obwohl die Saccharose-Synthase im Handel erhältlich ist und aktivierte Zucker sowie darüber erhältliche Di- und Oligosaccharide in der Zuckerchemie erhebliche Bedeutung besitzen.

Mono-, Oligo- und Polysaccharide haben vielfältige Funktionen als antigene Determinanten, bei der Zell-Zell-Erkennung, bei der Zelldifferenzierung und als Bindungsstellen für Toxine, Bakterien und Viren.

Eine zusammenfassende Darstellung von Herstellung und Anwendung findet sich bei S. David u.a. in Advances in Carbohydrate Chem. a. Biochem. 49 (1991) 175 - 237. Y. Ichikawa u.a. geben in Anal. Biochem. 202 (1992) 215 - 238 unterschiedliche Umsetzungsmechanismen an. Als "Large Scale Synthese" wird jedoch insbesondere auf die Umsetzung von Zucker-1-Phosphat (insbesondere Glucose-1-Phosphat) mit Nucleosidtriphosphat, insbesondere mit UTP, in Gegenwart von Pyrophosphorylase nach C.H. Wong u.a. (J. Org. Chem. 47 (1982) 5416 - 18) hingewiesen, die eine mehrstufige enzymatische Synthese von Nucleotidzuckern beschreiben. Diese Synthese nach C.H. Wong wird auch von Toone & Whiteside in Am. Chem. Soc. Sympos. Ser. 466 (1991) 1 - 22 als Methode der Wahl genannt.

Es wurde nun überraschenderweise festgestellt, daß Saccharose-Synthase-Isolate (insbesondere das im Handel erhältliche Enzym) allgemein mehr oder minder hohe Anteile an Nucleotidphosphatasen enthalten und daß deren Anwesenheit - auch in nur geringer Menge - die Synthese von NDP-Glucose und homologen Verbindungen so weit stört, daß die überzeugende Synthesetauglichkeit von Saccharose-Synthase bislang nicht erkannt werden konnte.

Durch angepaßte Reinigungsmethoden und empfindlichen Phosphatase-Nachweis wurde nunmehr eine Saccharose-Synthase entwickelt, die ausreichend stabil ist und eine glatte einstufige Synthesereaktion nach (1) ermöglicht.

Gegenstand des Erfindung ist ein Verfahren zur Herstellung von Saccharose-Synthase, dadurch gekennzeichnet, dass man einen Saccharose-Synthase enthaltenden Rohextrakt eines Folge von Reinigungsschritten unterwirft, die die Aufgabe eines ultrafiltrierten 50 mM KCL aufweisenden Extraktes von pH8 auf eine Sepharose-Q-Säule und eine Gradienten-Elution aus des Säule bei pH8 mit 50-500 mM KCL umfasst.

Im HPLC-Chromatogramm der aufgereinigten Saccharose-Synthase sind Nucleotid-phosphatasen nicht mehr nachweisbar (≤ 0,1 %).

Weitere Ausführungsformen der Erfindung ergeben sich aus den Patentansprüchen.

Als Quelle für die Saccharose-Synthase dienen insbesondere Reis, Mais oder Weizenkörner, die angequollen und mechanisch aufgeschlossen werden. Der dabei gewonnene wäßrige Rohextrakt wird entweder einer PEG-Fällung (A) oder einer Verteilung im wäßrigen 2-Phasensystem (B) unterworfen. Bei (A) kann eine fraktionierte Fällung vorgesehen werden, indem zunächst (A1) mit relativ niedermolekularem PEG (Polyethylenglykol; M ≥ 1000) und minderen PEG-Konzentrationen für die Ausfällung begleitender Proteine gesorgt wird (z.B. mit 5 % PEG 4000), während die Saccharose-Synthase im Überstand verbleibt, die dann in einem zweiten Schritt (A2) mit erhöhter PEG-Konzentration ausgefällt und aus dem Niederschlag mit 200 mM Hepes Puffer (pH 7,2) wieder herausgelöst wird. Die Fällung (A2) ist nicht unbedingt erforderlich und kann zur Vereinfachung des Verfahrens und Ausbeutesteigerung weggelassen werden, wie weiter unten gezeigt wird.

Das Molekulargewicht des PEGs bei den PEG-Fällungen kann mit entsprechender Änderung des PEG-Prozentsatzes variiert werden.

Die wieder in Lösung gebrachte Saccharose-Synthase bzw. der Überstand oder die enzymhaltige Phase der Extraktion wird zweckmäßigerweise nach einer Adsorption an Sephadex A50 und Stufenelution bei pH 7,2 (eingestellt mit Hepes-NaOH) mit 100 mM KCl und 300 mM KCl und Umpuffern sowie Ultrafiltration auf eine Sepharose-Q-Säule geladen und einer linearen Gradienten-Elution mit 50 - 500 mM KCl bei pH 8 (200 mM Hepes-NaOH) unterworfen und auf einer Gelfiltrationssäule chromatographiert.

Besonders wichtig ist dabei der Behandlungsschritt auf der Sepharose-Q-Säule mit Gradienten-Elution, wie angegeben. Auf diese Weise erhält man aufgereinigte Saccharose-Synthase, deren Nucleotidphosphatasegehalt < 0,1 %, d.h. im Phosphatase-Test der Enzym-Präparation nicht mehr nachweisbar ist.

Die erfindungsgemäß aufgereinigte Saccharose-Synthase läßt sich insbesondere zur enzymatischen Synthese von aktivierter Glucose und aktivierten Glucoseabkömmlingen durch Spaltung eines Di-, Tri-, Oligosaccharids oder deren Abkömmlingen mit Nukleosiddiphosphaten nutzen. Daraus resultierende Produkte, z.B. UDP-Glucose, TDP-Glucose und CDP-Glucose, sind wichtige Ausgangssubstrate für die enzymatische und/oder chemische Darstellung von aktivierten Desoxyzuckern und deren Abkömmlingen. Ein weiteres Beispiel für die oben genannten Anwendung ist die enzymatische Spaltung von 2-Desoxysaccharose mit Saccharose-Synthase unter Verwendung von Nukleosiddiphosphaten, z.B. UDP oder TDP.

In Kombination mit anderen Enzymen, z.B. UDP-Glucose Epimerase und Galaktosyltransferase, wird Saccharose-Synthase zur zyklischen Regenerierung von z.B. UDP-Glucose eingesetzt. So wird eine enzymatische Synthese eines Disaccarid-Abkömmiings wie z.B. N-Acetyllactosamin (LacNAc) mit 3 Enzymen durchgeführt. Im Vergleich zur publizierten enzymatischen Synthese von LacNAc (Wong u.a. J. Org. Chem. 47 (1982) 5416-5418) ergeben sich durch Reduktion der Anzahl der Enzyme wirtschaftliche Vorteile.

Die erfindungsgemäß aufgereinigte Saccharose-Synthase ist auch für die Synthese von Glucosiden sowie deren Abkömmlingen nützlich. So werden UDP-Glucose oder aktivierte Glucoseabkömmlinge auf Akzeptormoleküle mit mindestens einer Hydroxylgruppe übertragen. Beispiele für Zuckermoleküle als Akzeptoren sind bei den Ketosen die Isomeren der D-Fructose, z.B. D-Psicose, D-Tagatose und L-Sorbose sowie deren Abkömmlinge, z.B. 5,6-Didesoxy-5-keto-D-Fructose und 6-Desoxy-L-Sorbose. Beispiele für Zuckermoleküle als Akzeptoren bei den Aldosen sind L-Arabinose, D-Lyxose, D-Mannose sowie deren Abkömmlinge, z.B. 1,6-Anhydroglucose.

Di-, Tri- und Oligosaccharide sind ebenfalls Akzeptormoleküle, z.B. Lactulose, Isomaltulose und Raffinose. Andere hydroxylgruppen-haltige Akzeptormoleküle, die nicht zu der Substanzklasse der Zucker gehören, sind insbesondere heterocyclische Verbindungen mit mindestens einer Hydroxylgruppe am heterocyclischen Ring und/oder in einer daran befindlichen Seitenkette, z.B. 1-Ethyl-3-hydroxy-pyrollidin oder N-(2-Hydroxyethyl)piperidin.

Die nachfolgenden Beispiele zeigen die erfindungsgemäße Arbeitsweise im einzelnen. Dabei wird Bezug genommen auf die angefügten Zeichnungen; es zeigen:
- Fig. 1:: das Chromatogramm der Sepharose-Q-Trennung;
- Fig. 2:: die NDP-Glucose-Bildung mit unterschiedlichen Nucleotiden;
- Fig. 3 u. 4:: Die Bildung von TDP- und UDP-Glucose mit Saccharose-Synthase;
- Fig. 5 u. 6:: Reaktionsschemata für die enzymatische Synthese von N-Acetyllactosamin (nach Wong; Fig. 5 bzw. erfindungsgemäß; Fig. 6);
- Fig. 7 u. 8:: Nucleotid-Chromatogramme der Produktmischung (Schritt 1 u. 2 gemäß Fig. 6) nach Hitzeinaktivierung des Enzyms;
- Fig. 9:: das HPLC-Chromatogramm der Produktmischung (vollständiger Zyklus gemäß Fig. 6);
- Fig. 10 - 12:: Kurven zur Kinetik der Synthese-Reaktion (I);
- Fig. 13:: den Einfluß von Metallionen auf die Enzymaktivität;
und
- Fig. 14:: ein Diagramm für die Bildung von TDP-Glucose im EMR Enzymmembranreaktor (EMR).

### Beispiel 1: Isolierung der Saccharose-Synthase

800 g Reiskörner werden über Nacht in Hepes-NaOH Puffer pH 7,2 gequollen und anschließend im Waring Blender für 1,5 Min. aufgeschlossen. Nachdem mit einem Handmixer weitere 3 Min. homogenisiert worden ist, wird das Pellet abzentrifugiert (Sorvall GS3, 20 Min. 5000 rpm 4°C). Anschließend wird das Protein im Überstand mit PEG 4000 fraktioniert gefällt (5 und 20 % PEG). Das Pellet nach der 20 %igen PEG Fällung wird in Puffer aufgelöst und in einer Batch Adsorption an Sephadex A50 gebunden. 200 ml Sephadex-A50-Gel werden mit ca. 4 g Protein beladen. Die Stufenelution startet mit 300 ml Hepes-NaOH pH 7,2 und 300 ml Hepes-NaOH pH 7,2 mit 100 mM KCl. Das Enzym wird mit zwei Volumina (100 ml) Hepes-NaOH pH 7,2 mit 300 mM KCl eluiert. Nach Umpufferung und Ultrafiltration wird diese Fraktion auf eine Sepharose-Q-Säule (Hepes-NaOH pH 8,0 mit 50 mM KCl) geladen und mit einem linearen Gradienten (50 mM - 500 mM KCl in Hepes-NaOH 200 mM pH 8,0) eluiert. Die gesammelten Enzymfraktionen werden abschließend in einer Gelfiltrationssäule (Superdex 200 prep grade) chromatographiert.

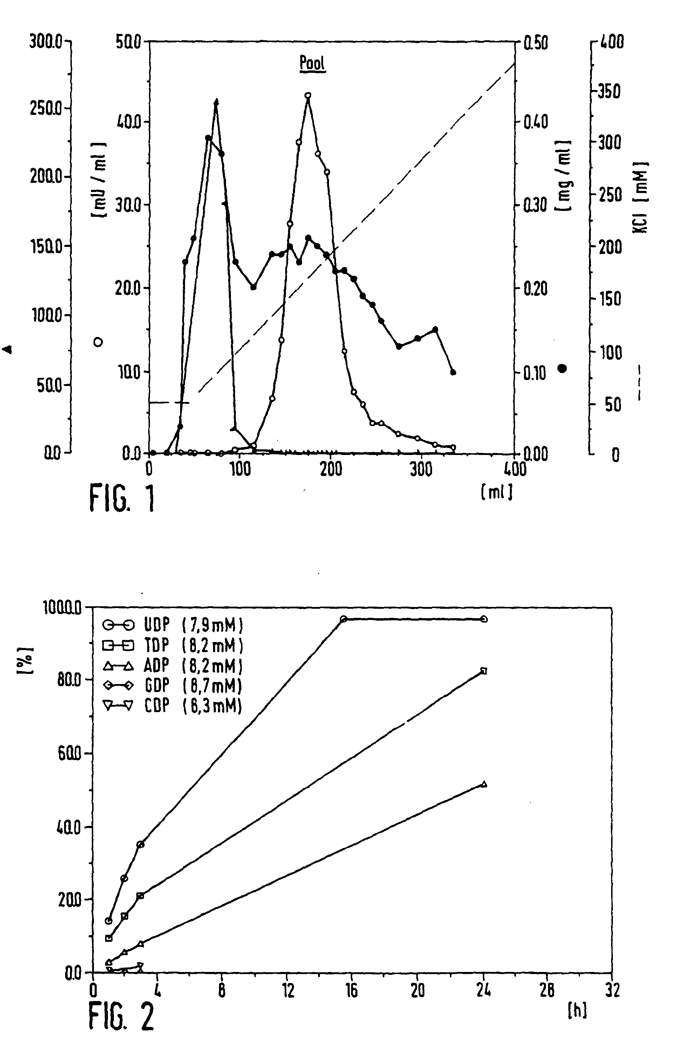

Saccharose-Synthase aus Reis wurde 151-fach mit einer Ausbeute von 5,4 % angereichert (Tabelle 1 A). Ein großer Verlust entsteht bei der Fällung mit Polyethylenglykol 4000 (ca. 80 % Verlust bei der 5 - 20 % Fällung). Alternativ kann eine Enzymanreicherung aus dem Rohextrakt mit Hilfe eines wäßrigen 2-Phasensystems (PEG/Salz) vorgesehen werden. Sehr effektiv sind die Batch Adsorption an Sephadex A50 und die anschließende Anionenchromatographie an Sepharose-Q-FastFlow mit einem Aufreinigungsfaktor von insgesamt 43. Außerdem wird die Nukleotiddi- und -monophosphatatse-Aktivität vollständig abgetrennt (Fig. 1), was für den Einsatz der Saccharose-Synthase in enzymatischen Synthesen sehr wichtig ist.

Das Molekulargewicht des nativen Enzyms beträgt 362000 ± 7000 Da, es besteht aus 4 Untereinheiten zu je 90000 Da und besitzt keine inter- oder intramolekularen Disulfidbrücken. Die N-terminale Aminosäuresequenzierung durch automatisierten Edman-Abbau in einem Pulsed Liquid Protein Sequencer erbrachte, daß der N-Terminus der Untereinheit blockiert ist. Der isoelektrische Punkt des nativen Enzyms liegt bei pH 6,16.

Die Reinigung der Saccharose-Synthase wurde hinsichtlich der eingesetzten Menge Reis und der Anzahl der Reinigungsschritte optimiert. Tabelle 1 B zeigt, daß die Ausbeute bei nur geringfügig geringerer Reinheit auf 21 % gesteigert wurde. Die Reinigung umfaßt statt sechs nur noch vier Schritte, wobei die Fällung bei 20 % PEG 4000 und die Batch Adsorption auf Sephadex A50 entfallen. Das gereinigte Enzym ist auch nach dieser Reinigung frei von Nukleotidmono- und -diphosphatasen.

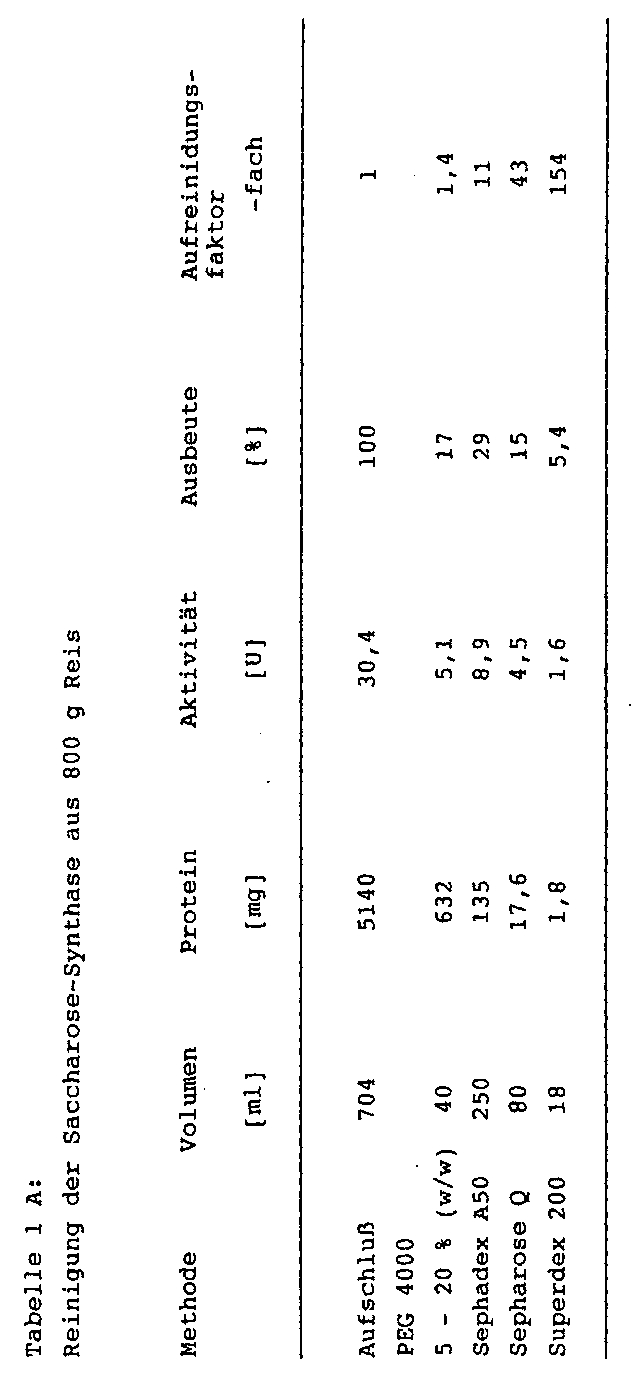

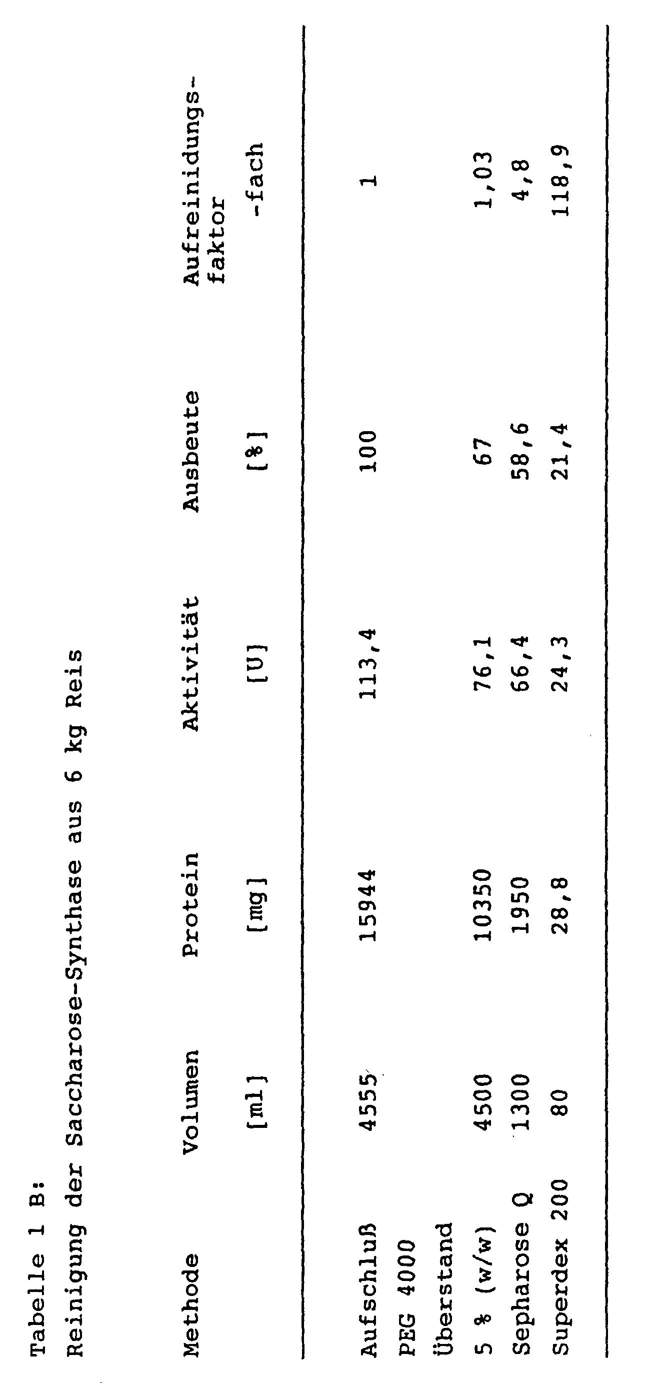

Die Untersuchung der Spalt- und Synthese-Reaktion mit Saccharose-Synthase läßt erkennen, daß
1. Saccharose-Synthase zur enzymatischen Synthese von UDP-Glucose, TDP-Glucose, GDP-Glucose und CDP-Glucose aus Saccharose geeignet ist.
2. die Kombination der Saccharose-Synthase mit anderen Enzymen (s.o.) zur enzymatischen Synthese von sekundären Nukleotidzuckern (UDP-Galactose, UDP-Glucuronsäure) genutzt werden kann.
3. bei der enzymatischen Synthese von Oligosacchariden im Enzymmembranreaktor der Einsatz der Saccharose-Synthase zur "Kofaktorregenerierung" eine erhebliche Vereinfachung der kinetischen Kontrolle darstellt.
4. das Substratspektrum der Saccharose-Synthase für Di-, Triund Oligosaccharide sowie Glykoside zu bisher noch nicht zugänglichen aktivierten Mono-, Di- und Oligosacchariden führt.
5. andere Nukleotidzucker als UDP-Glucose in der Synthesereaktion mit Fructose eingesetzt werden können.
6. Fructose durch andere Zucker mit β-Furanosekonfiguration, sowie durch Zuckeralkohole und andere chemische Verbindungen mit strukturellen Ähnlichkeiten zur β-Furanose, ersetzt werden kann.

Nachfolgend werden Beispiele für die Wirkungsweise und Anwendung der Saccharose-Synthase beschrieben:

### 1. Substratspektrum der Nukleosiddiphosphate

Es wurden UDP, TDP, CDP, ADP und GDP untersucht. Die Reaktionsansätze enthielten:
550 µl Hepes-NaOH (200 mM, pH 7,2)
250 µl Saccharose (2 M)
100 µl Nukleosiddiphosphat (15 - 90 mM)
100 µl gereinigte Saccharose-Synthase (21,1 mU/ml)

Die Reaktionsansätze wurden bei 30°C inkubiert und zu verschiedenen Zeiten abgestoppt (5 min. bei 95°C). Nach Filtration der Probe durch einen 0,22 µm Filter wurden die entstandenen Nukleotidzucker mittels Ionenpaar HPLC analysiert.

Die Bildung von UDP- und TDP-Glucose wurde an Hand von Eichkurven für die HPLC Chromatogramme (Peak Area/Konzentration) quantifiziert.

Figur 2 zeigt, daß die Nukleosiddiphosphate in der Reihenfolge UDP, TDP, ADP, CDP und GDP akzeptiert werden. Das gereinigte Enzym war frei von Nukleotidphosphatasen (NPasen, Kontrolle ohne Saccharose), die Nukleosiddiphosphate zu den -monophosphaten oder auch Basen abbauen. Im HPLC Chromatogramm konnten die auftretenden Peaks für UMP, TMP, Uridin und Thymidin durch geeignete Kontrollversuche auf Verunreinigungen im Substrat und auf die Zersetzung der Nukleosiddiphosphate durch die Hitzebehandlung zurückgeführt werden. Nach Hitzebehandlung von 1,57 mM UDP entstanden 0,123 mM UMP; davon waren 0,082 mM (5 %) als Verunreinigungen im UDP-Substrat bereits vorhanden.

Aus einer Untersuchung des zeitlichen Konzentrationsverlauf der Synthese von UDP- und TDP-Glucose wird deutlich, daß durch steigende Mengen des Enzyms die nahezu vollständige Umsetzung der Nukleosiddiphosphate zu Nukleotidzucker in kurzen Reaktionszeiten erreicht werden kann.

Tabelle 2 faßt die Umsatzraten für die Synthese von UDP- und TDP-Glucose zusammen. Mit 0,16 mU Enzym wird die Raum-Zeit-Ausbeute bei höheren Substratkonzentrationen nur wenig gesteigert. Mit 1,8 mU Saccharose-Synthase wird nach 3 h ein 99 %iger Umsatz bezogen auf die eingesetzte UDP-Konzentration (1,57 mM) erreicht. Das entspricht einer Raum-Zeit-Ausbeute von 0,21 g/l*h. Mit einer höheren UDP-Konzentration (7,86 mM) beträgt die Raum-Zeit-Ausbeute 0,4 g/l*h.

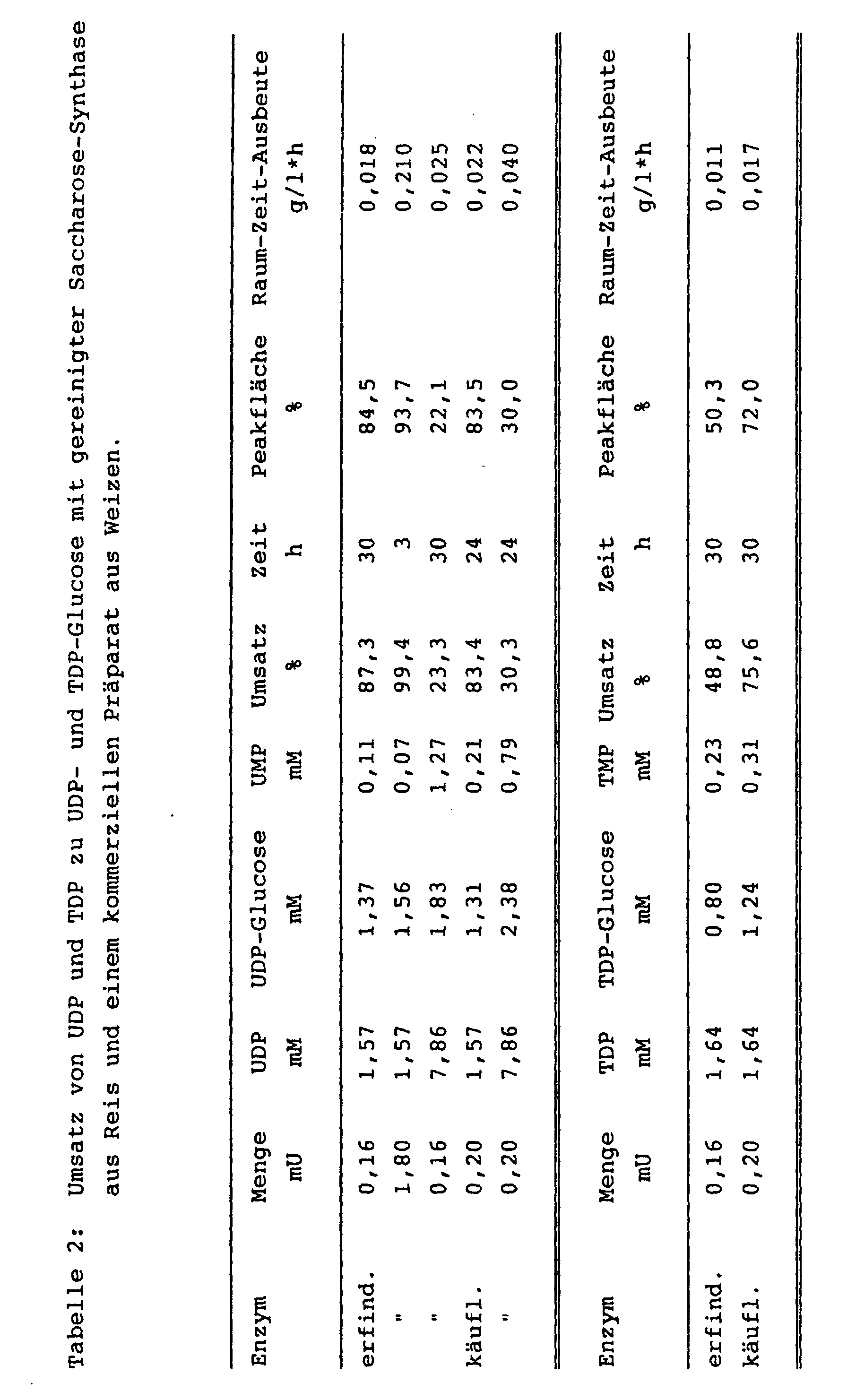

Die Raum-Zeit-Ausbeute für die Synthese von TDP-Glucose beträgt für 0,16 mU eingesetztes Enzym 0,011 g/l*h; das entspricht einem 49 %igen Umsatz nach 30 h für 0,16 mU Enzym. Ein weiteres Experiment zeigt, daß der Umsatz von 8,2 mM TDP mit 0,2 mU Enzym nach 24 h auf 80 % gesteigert werden kann.

Zum Vergleich wurde ein kommerzielles Präparat der Saccharose-Synthase aus Weizen (spezifische Aktivität 8,15 mU/mg) getestet. Dieses Enzym zeigt ähnliche Raum-Zeit-Ausbeuten wie das gereinigte Enzym aus Reis. Allerdings zeigen die HPLC Chromatogramme die gleichzeitige Bildung relativ großer Mengen UMP und Uridin durch Nukleotidphosphatasen (Tabelle 2). Diese Enzymverunreinigungen sind im gereinigten Enzym aus Reis nicht mehr vorhanden. Die auftretenden UMP- und Uridin-Peaks im Chromatogramm sind allein auf Erhitzung der Proben zurückzuführen.

### 2. Puffer Spektrum

Das Pufferspektrum für die Synthese von UDP- und TDP-Glucose mit der gereinigten Saccharose-Synthase aus Reis und dem kommerziellen Enzym aus Weizen wurde untersucht. Folgende Puffer wurden getestet (alle 200 mM und pH 7,2, angegeben sind die pH-Pufferbereiche):

| | |
|---|---|
| Mops-NaOH-NaCl | pH 6,25 - 8,15 |
| TES-NaOH-NaCl | pH 6,55 - 8,45 |
| Tris-HCl | pH 7,00 - 9,00 |
| Hepes-NaOH | pH 6,80 - 8,20 |
| KH₂PO₄-NaOH | pH 5,80 - 8,00 |
| Na₂HPO₄-NaH₂PO₄ | pH 5,80 - 8,00 |
| Imidazol-HCl | pH 6,20 - 7,80 |
| TEA-Hydrochlorid - NaOH | pH 6,80 - 8,80 |

Die Inkubationsansätze enthielten:
550 µl Puffer (200 mM, pH 7,2)
250 µl Saccharose (2 M)
100 µl UDP (15,7 mM) oder TDP (16,4 mM)
100 µl Enzymlösung

Inkubation und Analytik wurden wie unter 1. beschrieben durchgeführt.

Es zeigt sich, daß der bisher benutzte Hepes-NaOH-Puffer für die Synthese von UDP- und TDP-Glucose am besten geeignet ist. Für beide Enzyme ergeben der Mops- und der TES-Puffer 60 - 80 % Restaktivität. Das kommerzielle Enzym zeigt im Gegensatz zum Reisenzym im TEA-Puffer eine um 30 - 40 % höhere Restaktivität. In den restlichen Puffern haben beide Enzyme eine Restaktivität von unter 50 %.

Dieses Ergebnisse zeigen, daß die Wahl des Puffers Auswirkungen auf die Aktivität der Saccharose-Synthase hat und die Bestimmung des pH-Optimums beeinflussen kann.

### 3. pH-Optimum

Folgende Puffer wurden für die Bestimmung des pH-Optimums verwendet (alle 200 mM):

| | |
|---|---|
| Na-Citrat-Citronensäure | pH 4,0 - 6,2 |
| KH₂PO₄-NaOH | pH 5,8 - 7,2 |
| Mops-NaOH-NaCl | pH 6,3 - 7,4 |
| Hepes-NaOH | pH 6,8 - 8,2 |
| TEA-Hydrochlorid - NaOH | pH 7,2 - 8,8 |

Die Inkubationsansätze enthielten:
640 µl Puffer (200 mM)
250 µl Saccharose (2 M)
100 µl UDP (15,7 mM) oder TDP (16,4 mM)
10 µl Enzymlösung

Inkubation und Analytik erfolgten wie unter 1. beschrieben.

Beide Enzyme zeigen in Abhängigkeit von den verwendeten Puffern unterschiedliche pH-Optima.

Mit UDP als Substrat haben beide Enzyme bei Verwendung von Citrat- oder Phosphat-Puffer ein pH-Optimum zwischen pH 5,5 und 5,7.

Bei Verwendung von Hepes- und Mops-Puffer liegt das Optimum für die UDP-Glucose-Synthese zwischen pH 6,7 und 7,0.

Für die Synthese von TDP-Glucose gilt dies in gleicher Weise; mit Citrat- oder Phosphat-Puffer liegt das Optimum zwischen pH 5,8 und 6,2 und mit Mops- oder Hepes-Puffer zwischen pH 6,5 und 6,8.

### 4. pH-Stabilität

Für die Ermittlung der pH-Stabilität wurde das Reisenzym bei verschiedenen pH-Werten in Hepes-NaOH-Puffer (200 mM) und Raumtemperatur verschieden lang inkubiert. Das Enzym wurde anschließend in den herkömmlichen Aktivitätstest eingesetzt:
550 µl Hepes-NaOH (200 mM, verschiedene pH-Werte)
250 µl Saccharose (2 M)
100 µl UDP (20 mM)
100 µl Enzymlösung

Nach 1 h Reaktionszeit wurde die Probe wie oben beschrieben mit HPLC analysiert.

Die gereinigte Saccharose-Synthase aus Reis zeigt bei pH 7,0 und 7,9 nach 2 Tagen eine Restaktivität von > 60 %, die sich für die Synthese von UDP- und TDP-Glucose ausnutzen lassen.

### 5. Temperaturoptimum

Zur Bestimmung des Temperaturoptimums wurde das Reisenzym 1 h bei pH 6,5 (pH-Optimum) und verschiedenen Temperaturen inkubiert. Anschließend wurde das Enzym in folgenden Aktivitätstest eingesetzt:
550 µl Puffer (200 mM, pH 6,5)
250 µl Saccharose (2 M)
100 µl UDP (20 mM)
100 µl Enzymlösung

Nach 1 h Reaktionszeit wurde die Probe wie oben beschrieben mit HPLC analysiert. Zusätzlich wurde jeweils eine Kontrolle ohne Enzym inkubiert und analysiert.

Für die Spaltung von Saccharose mit UDP liegt das Temperaturoptimum der Saccharose-Synthase aus Reis bei pH 6,5 zwischen 50° und 60°C.

### 6. Temperatur-Stabilität

Das Enzym besitzt nach 5 h bei 37°C seine volle Aktivität, nach 5 h bei 56°C ist eine Restaktivität von 37 % vorhanden.

### 8. Kinetik

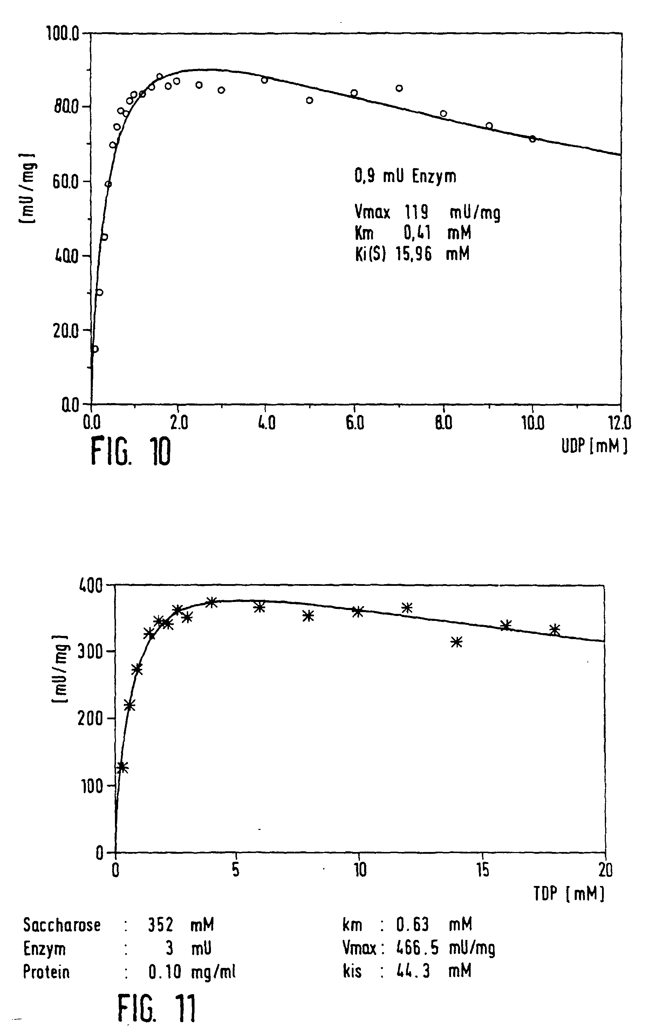

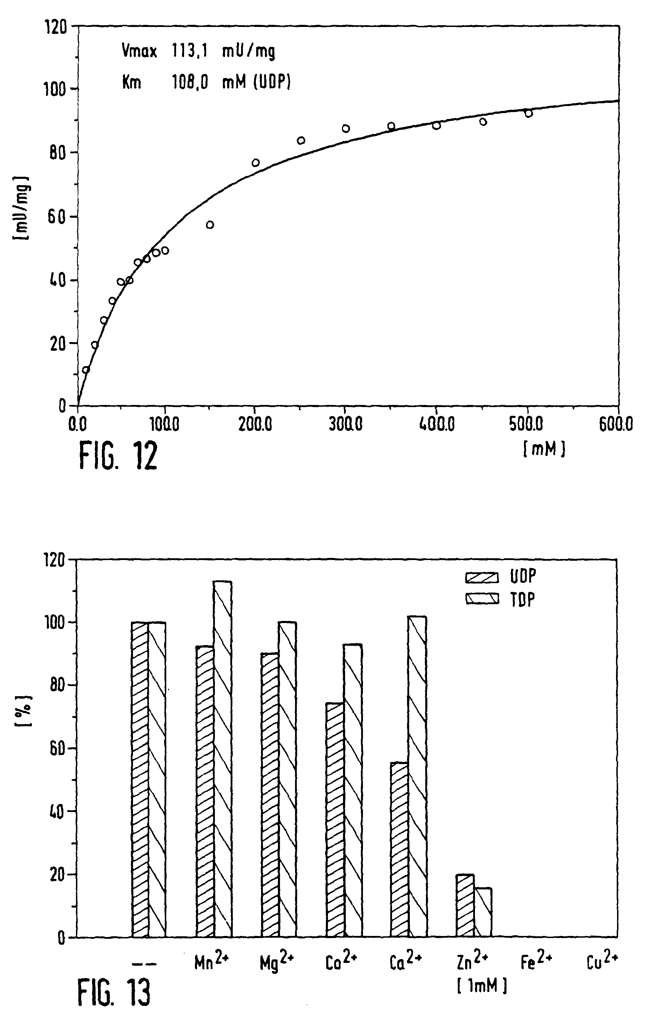

Zur Bestimmung von Vₘₐₓ und Kₘ der Substrate wurden UDP oder TDP bei konstanter Saccharose-Konzentration von 500 mM zwischen 0 und 10 mM im Reaktionsansatz variiert. Bei konstanter UDP (2 mM) Konzentration wurde Saccharose zwischen 0 und 500 mM im Reaktionsansatz variiert. Alle Reaktionsansätze wurden 1 h bei 30°C und pH 6,5 (Hepes-NaOH 200 mM) inkubiert. Die Proben wurden wie oben beschrieben behandelt und mit HPLC analysiert. Das Reisenzym zeigt für UDP eine Substratüberschußinhibition (Ki(S) = 16 mM mit 0,9 mU Enzym) bei einem Km-Wert von 0,4 mM (Figur 10). Der Km-Wert für TDP beträgt 0,65 mM (Figur 11). Der Substratüberschußinhibition kann durch höhere Enzymmengen entgegengewirkt werden. Der Km-Wert für Saccharose beträgt 108 mM (Figur 12).

### 9. Abhängigkeit von zweiwertigen Metallionen

Figur 13 zeigt, daß in Anwesenheit von 1 mM Metallionen, wie z.B. Mn²⁺ und Mg²⁺ die Aktivität der Saccharose-Synthase nur wenig beeinflußt wird. Eine Stimulierung der Enzymaktivität tritt durch Mn²⁺ und Ca²⁺ mit TDP als Substrat auf. In Anwesenheit von Cu²⁺ und Fe²⁺ wird das Enzym vollständig inaktiviert.

### 10. Enzymatische Synthese von UDP- und TDP-Glucose unter optimierten Bedingungen

Die Reaktionsansätze enthielten:
550 µl Hepes-NaOH (200 mM, pH 7,2)
250 µl Saccharose (2 M)
100 µl Nukleosiddiphosphat (UDP 100 mM oder TDP 124 mM)
100 µl gereinigte Saccharose-Synthase (15 mU/ml)

Die Reaktionsansätze wurden mit UDP bei pH 7,0 bzw. mit TDP bei pH 6,8 bei 30°C inkubiert und zu verschiedenen Zeiten abgestoppt (5 min. bei 95°C). Nach Filtration der Probe durch einen 0,22 µm Filter wurden die entstandenen Nukleotidzucker mittels Ionenpaar HPLC analysiert.

Die Bildung von UDP- und TDP-Glucose wurde an Hand von Eichkurven für die HPLC-Chromatogramme (Peak Area/Konzentration) quantifiziert.

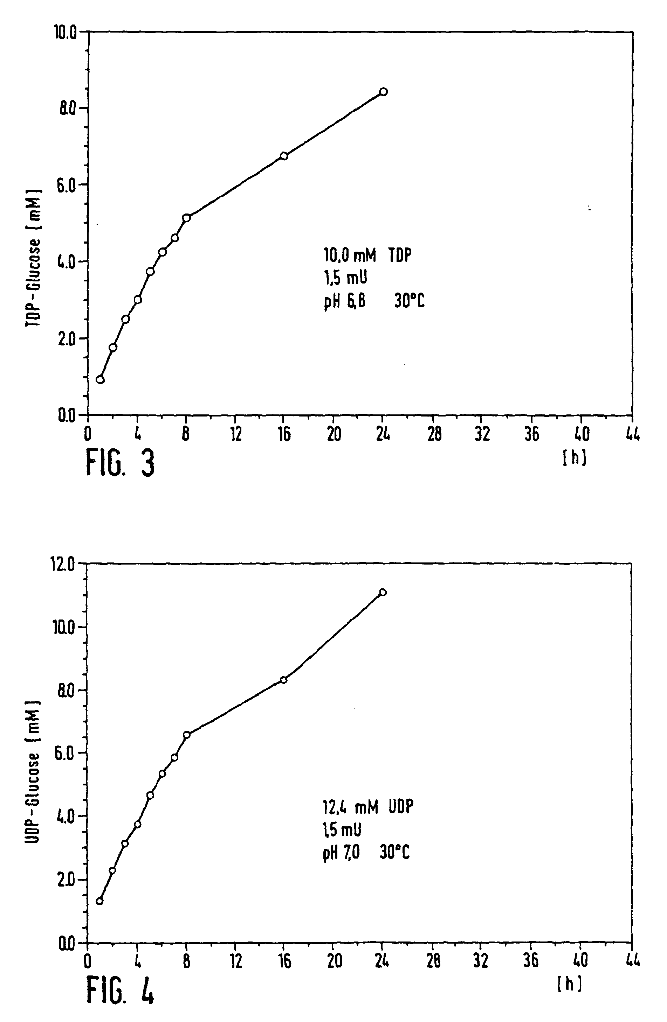

Figur 3 und 4 zeigen, daß nach 24 h 92 % des UDP zu UDP-Glucose und 84 % des TDP zu TDP-Glucose umgesetzt wurden.

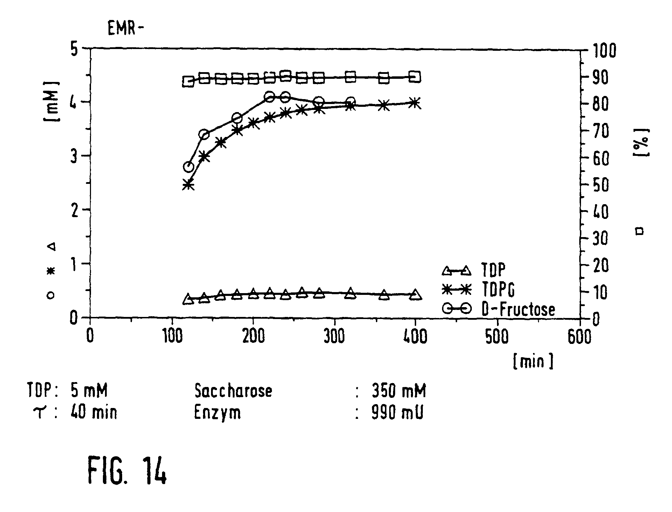

Die gereinigte Saccharose-Synthase wurde zur enzymatischen Synthese von TDP-Glucose im Enzymmembranreaktor (10 ml Reaktorvolumen) eingesetzt. Figur 14 zeigt den Umsatz von TDP zu TDP-Glucose und die Konzentrationen von TDP, TDP-Glucose und Fructose bei 5 mM TDP, 350 mM Saccharose, 40 Minuten Verweilzeit und 990 mU Saccharose-Synthase. Der Umsatz beträgt 89,6 % bezogen auf das eingesetzte TDP. Die theoretische Raum-Zeit-Ausbeute für ein Liter Reaktorvolumen ergibt 98,1 g TDP-Glucose pro Liter und Tag.

### 11. Substratspektrum der Saccharose-Synthase für die Spaltreaktion (I)

Für die Spaltreaktion der Saccharose-Synthase wurde Saccharose gegen andere Di- oder Trisaccharide ausgetauscht:

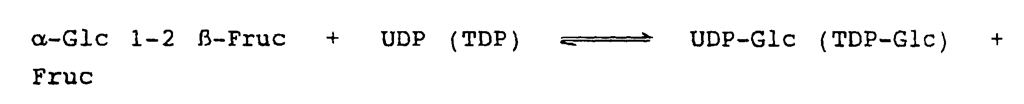

Die Konzentration der Saccharide betrug 75 bis 500 mM im Reaktionsansatz. Es wurden 2 mM UDP oder TDP und in der Regel 10-80 mU Enzym eingesetzt. Nach 3 h bei 30°C und pH 7,2 (200 mM Hepes-NaOH) wurde die Reaktion bei 95°C 5 min. abgestoppt. Die Bildung von Nukleotidzuckern wurde mit HPLC verfolgt und mit einer Kontrolle (ohne Enzym) verglichen.

Tabelle 3 zeigt, daß das Disaccharid Isomaltulose (Palatinose) und die Trisaccharide Raffinose und Melezitose anstelle der Saccharose umgesetzt werden können.

### 12. Substratspektrum der Saccharose-Synthase aus Reis für die Synthese von Disacchariden

### A: Variationen der Nukleotidzucker

Für die Synthesereaktion wurden die Nukleotidzucker variiert:

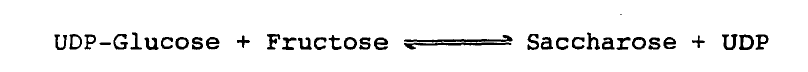

Es wurden zunächst nur UDP-aktivierte Zucker eingesetzt: UDP-Galactose, UDP-N-Acetylglucosamin, UDP-Glucuronsäure, UDP-N-Acetylgalactosamin.

Die Ansätze enthielten:
550 µl Puffer (200 mM, pH 7,5)
250 µl Fructose (40 mM)
100 µl UDP-Zucker (20 mM)
100 µl Enzymlösung

Die Ansätze wurden 2 h bei 30°C inkubiert und 5 min. bei 95°C gestoppt. Mit HPLC wurde nach Vergleich mit einer Kontrolle (ohne Enzym) die Entstehung von UDP verfolgt.

Neben UDP-Glucose kann auch UDP-N-Acetylglucosamin und UDP-Xylose vom Reisenzym umgesetzt werden (Tabelle 4).

**Tabelle 3:**

| Überprüfung der Substratspaltung mit gereinigter Saccharose-Synthase aus Reis und UDP zur Synthese von aktivierten Zuckern. | | |
|---|---|---|
| Name | Bindung | Relative Aktivität % |
| Saccharose | α Glc 1-2 β Fruc | 100 |
| Saccharose-6'-P | | - |
| 2-Desoxy-Saccharose | | 55 |
| Turanose | α Glc 1-3 β Fruc | - |
| Isomaltulose | α Glc 1-6 β Fruc | 1,0 |
| Lactulose | β Gal 1-4 Fruc | - |
| Trehalose | α Glc 1-1 α Glc | - |
| Maitose | α Glc 1-4 Glc | - |
| Isomaltose | α Glc 1-6 Glc | - |
| Laminaribose | β Glc 1-3 Glc | - |
| Cellobiose | β Glc 1-4 Glc | - |
| β-Gentiobiose | β Glc 1-6 Glc | - |
| Mannobiose | α Man 1-3 Man | - |
| N'N'Diacetylchitobiose | β GlcNAc 1-4-GlcNAc | - |
| β-Lactose | β Gal 1-4 β Glc | - |
| α-Lactose | β Gal 1-4 α Glc | - |
| α-D-Melibiose | α Gal 1-6 Glc | - |
| LacNAc | β Gal 1-4 GlcNAc | - |
| Ampicillin | | 2,4 |
| Chlorogenic acid | | - |
| Thiodigalactosid | | - |
| Thiodiglucosid | | - |
| p-Aminophenyl-β-L-Fucopyranosid | | - |
| 3-0-β-D-Galactopyranosyl-D-Arabinose | | - |
| Octyl-β-D-Glucopyranosid | | - |
| p-Aminophenyl-β-D-galactopyranosid | | - |
| Raffinose | α Gal 1-6 α Glc 1-2 β Fruc | 3,8 |
| Melizitose | α Glc 1-3 β Fruc 2-1 α Glc | 0.4 |

**Tabelle 4:**

| Substratspektrum der gereinigten Saccharose-Synthase aus Reis. Es wurden verschiedene UDP-Zucker mit Fructose als Akzeptor eingesetzt. | |
|---|---|
| Name | Relative Aktivität % |
| UDP-Glucose | 100 |
| UDP-Galactose | - |
| UDP-N-Acetylglucosamin | 1,8 |
| UDP-N-Acetylgalaktosamin | - |
| UDP-Glucuronsäure | - |
| UDP-Xylose | 1,7 |

### 12. Substratspektrum der Saccharose-Synthase aus Reis für die Synthese von Disacchariden

### B: Variation des Akzeptors

Für die Synthesereaktion wurde der Akzeptor variiert. Neben dem natürlichen Akzeptor wurden andere Diastereomere der D-Fructose, wie D-Psicose, D-Tagatose, D-Sorbose eingesetzt. Weiterhin wurden systematisch einige Ketosen getestet. Neben Aldosen wurden auch Nichtzuckerakzeptoren untersucht. Tabelle 5 verdeutlicht, daß die untersuchten Diastereomere der D-Fructose bis auf D-Sorbose alle akzeptiert werden. L-Sorbose, D-Xylulose und die aufgeführten Desoxyketosen sind ebenfalls Akzeptoren der Saccharose-Synthase. Von den Aldosen werden D-Mannose, D-Lyxose und L-Arabinose akzeptiert. Als Akzeptoren können auch Derivate der Glucose fungieren, z.B. 1,6 Anhydro-β-D-Glucose oder Octyl-β-D-Glucopyranosid.
Di- und Trisaccharide (z.B. Lactulose oder Raffinose) können ebenfalls als Akzeptoren dienen. Von den Nichtzuckerakzeptoren können Derivate des Pyrrolidins in der Synthesereaktion der Saccharose-Synthase eingesetzt werden.

**Tabelle 5:**

| Substratspektrum der gereinigten Saccharose-Synthase aus Reis. Es wurden verschiedene Akzeptorsubstrate mit UDP-Glucose umgesetzt. | |
|---|---|
| Name | Relative Aktivität % |
| D-Fructose | 100 |
| Sedoheptulose Anhydrid | 1,1 |
| Mannoheptulose | - |
| D-Psicose | 14,1 |
| D-Tagatose | 28,3 |
| D-Sorbose | - |
| L-Sorbose | 6,0 |
| D-Ribulose | - |
| D-Xylulose | 40,7 |
| L-Xylulose | - |
| D-Erythrulose | - |
| 5-Keto-6-desoxy-D-fructose | 25,1 |
| 5,6-Didesoxy-5-methyl-D-fructose | 19,0 |
| 5,6-Didesoxy-D-fructose | 7,6 |
| 6-Desoxy-L-sorbose | 8,8 |
| D-Glucoheptose | - |
| β-D-Allose | - |
| β-L-Allose | - |
| D-Altrose | - |
| D-Glucose | - |
| L-Glucose | - |
| D-Mannose | 3,9 |
| L-Mannose | - |
| L-Gulose | - |
| D-Idose | - |
| L-Idose | - |
| D-Galactose | - |
| L-Galaktose | - |
| α-D-Taiose | - |
| D-Ribose | - |
| L-Ribose | - |
| D-Arabinose | - |
| L-Arabinose | 3,2 |
| D-Xylose | - |
| L-Xylose | - |
| D-Lyxose | 14,7 |
| D-Sorbit | - |
| D-Arabit t | - |
| L-Arabit | - |
| L-Ascorbinsäure | - |
| 2-Desoxy-Glucose | - |
| 1,6-Anhydroglucose | 9,1 |
| n-Octyl-β-D-glucopyranosid | 0,5 |
| Hydroxypyruvat t | - |
| 3-Hydroxybenzaldehyd | - |
| 3-Hydroxy-tetrahydrofuran | - |
| Tetrahydro-3-furan-methanol | - |
| S(+)-2-(Hydroxymethyl)-pyrrolidin | - |
| 3-Hydroxypyrrolidin | 6,3 |
| 3-Hydroxy-N-methyl-pyrrolidin | 1,9 |
| 1-Ethyl-3-hydroxy-pyrrolidin | 10,4 |
| 3-Pyrrolidino-1,2-propandiol | 9,6 |
| N-(2-Hydroxymethyl)piperidin | 9,4 |
| Tropin | 10,3 |
| | |
| Turanose | 0,5 |
| Lactulose | 12,3 |
| Raffinose | 8,4 |
| Isomaltulose | 3,1 |
| β-Lactose | - |
| Melizitose | 0,6 |

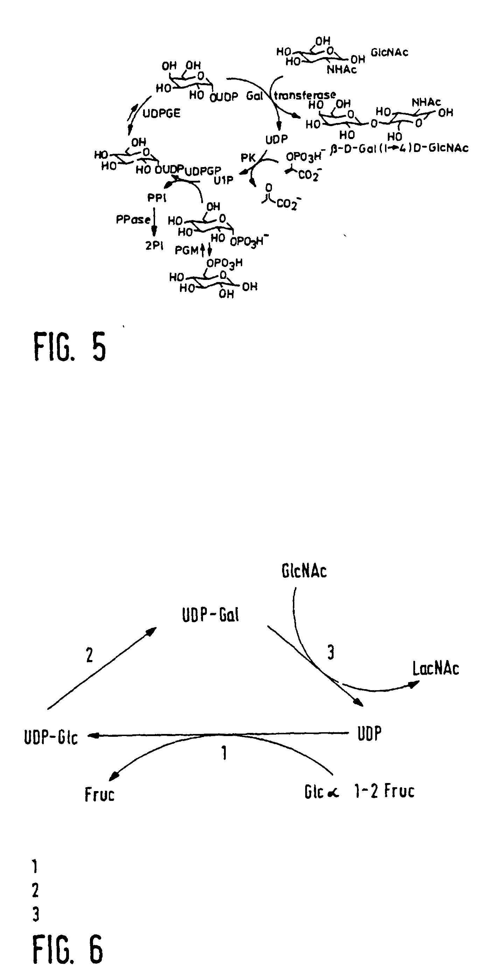

### 13. Enzymatische Synthese von UDP-Galaktose und N-Acetyllactosamin nach Schema gemäß Fig. 6

UDP-Galaktose und N-Acetyllactosamin wurden gemäß Fig. 6 hergestellt (Fig. 5 zeigt Synthesezyklus nach Wong)

### Ansatz:

677 µl Hepes-Puffer (50 mM, pH 7)
100 µl UDP (100 mM)
100 µl Saccharose
123 µl Enzym (10 mU im Ansatz)

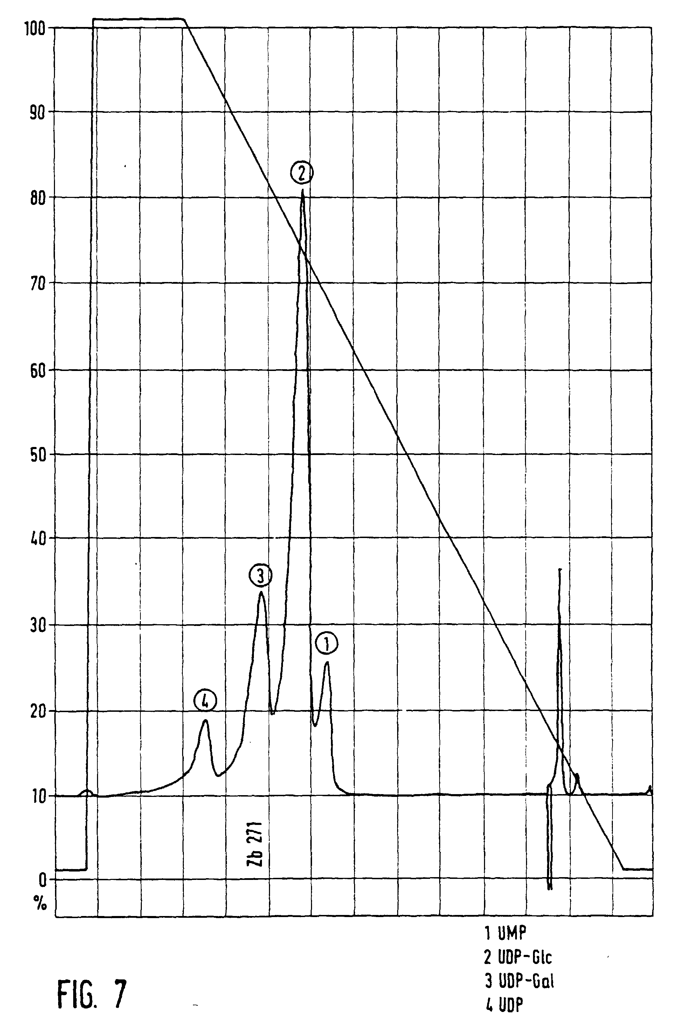

Nach 3 h Inkubation bei 30°C waren 80 % des UDP zu UDP-Glucose laut HPLC-Analyse umgesetzt. Anschließend wurden 100 mU UDP-Galaktose-Epimerase zugegeben und über Nacht bei 30°C inkubiert. Figur 7 zeigt, daß UDP-Galaktose aus UDP-Glucose entstanden ist. Da das Gleichgewicht der UDP-Galaktose-Epimerase stark auf der Seite von UDP-Glucose liegt, können UDP-Glucose/UDP-Galaktose Verhältnisse von 0,3 erwartet werden (siehe Peakhöhenverhältnisse von UDP-Glucose/UDP-Galaktose in Fig. 7).

UDP-Galaktose kann auch enzymatisch hergestellt werden, indem die benötigten Enzyme gleichzeitig im Ansatz inkubiert werden.

### Ansatz:

500 mM Saccharose
1-10 mM UDP
3-30 mU Saccharose-Synthase
200 mU UDP-Gal-Epimerase
alles in 200 mM Hepes-Puffer pH 7,2

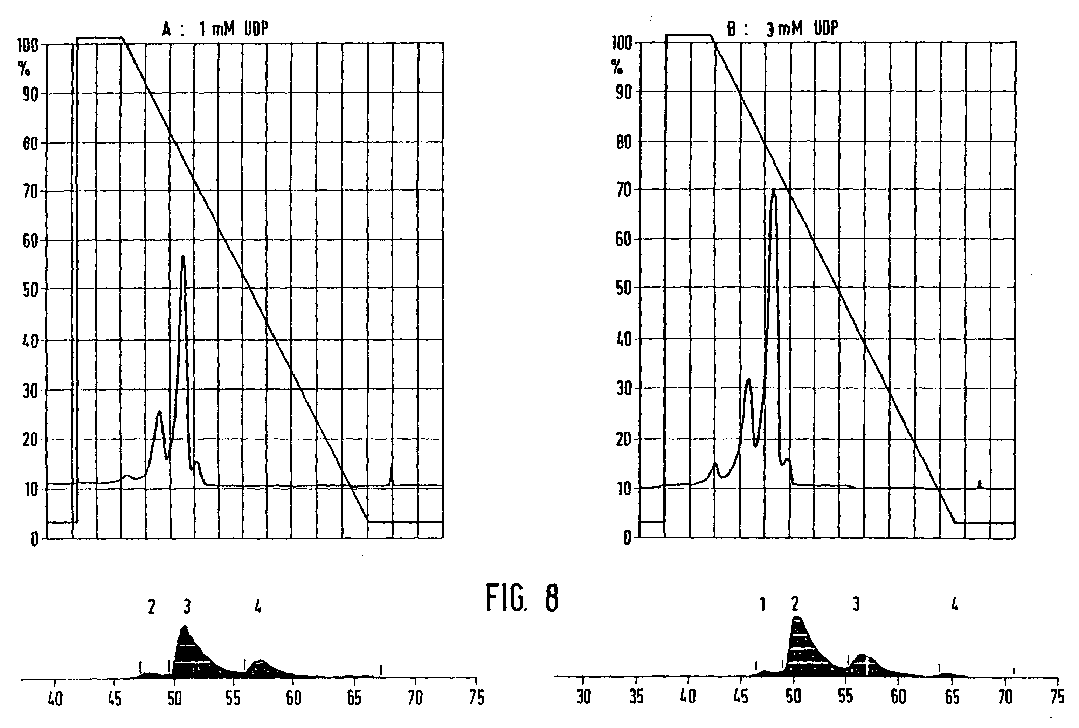

Die Ergebnisse sind Figur 8 dokumentiert.

Zur enzymatischen Synthese von N-Acetyllactosamin wurde folgender Versuch durchgeführt.
1 mM UDP
1 mM MnCl₂
5 mM N-Acetylglucosamin
500 mM Saccharose
200 mU UDP-Gal-Epimerase
100 mU β-1,4-Galaktosyltransferase
120 mU Saccharose-Synthase
alles in 200 mM Hepes-NaOH-Puffer pH 7,2 bei 30°C über Nacht inkubiert.

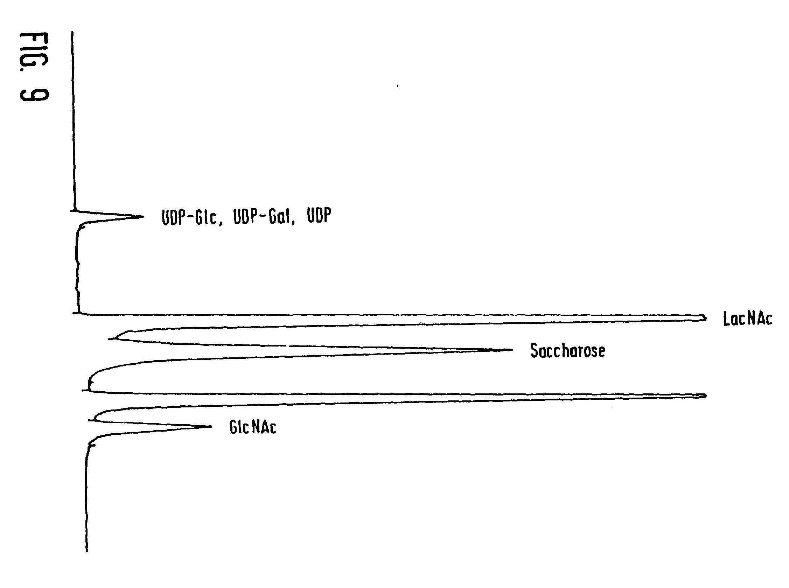

Figur 9 zeigt, daß N-Acetyllactosamin mit Hilfe von drei Enzymen (Figur 6) gebildet wird. Der Umsatz beträgt 80 % bezogen auf die eingesetzte Konzentration an N-Acetylglucosamin.

Im Rahmen der vorliegenden Untersuchungen wurde aus Reiskörnern isolierte Saccharose-Synthase verwendet, jedoch kann auch aus Weizen o.a. unter Beachtung der erfindungsgemäßen Vorschriften eine für die einstufige Bildung aktivierter Monosaccharide brauchbare Saccharose-Synthase erhalten werden.

## Patentansprüche

1. Verfahren zur Herstellung von Saccharose-Synthase
**dadurch gekennzeichnet,**
**daß** man einen Saccharose-Synthase enthaltenden Rohextrakt einer Folge von Reinigungsschritten unterwirft, die die Aufgabe eines ultrafiltrierten 50 mM KCl aufweisenden Extraktes von pH 8 auf eine Sepharose-Q-Säule und eine Gradienten-Elution aus der Säule bei pH 8 mit 50 -500 mM KCl umfaßt.

2. Verfahren nach Anspruch 1,
**dadurch gekennzeichnet,**
**daß** man einen Rohextrakt aus angequollenen und mechanisch aufgeschlossenen Reiskörnern folgenden Reinigungsschritten unterwirft:
- Ausfällung begleitender Proteine mit PEG oder wäßrige 2-Phasentrennung;
- Aufgabe des auf pH 8 eingestellten Überstandes bzw. der entsprechend eingestellten, die Enzymaktivität enthaltenden Phase auf eine Sepharose-Q-Säule;
- Gradienten-Elution bei pH 8 mit 50 -500 mM KCl; und
- Gelfiltrationssäulen-Chromatographie.

3. Verfahren nach Anspruch 1 oder 2,
**dadurch gekennzeichnet,**
**daß** die so gewonnene Saccharose-Synthase zur Umsetzung von Nucleosiddiphosphaten mit Di, Tri, oder Oligosacchariden bzw. Abkömmlingen derselben unter Bildung von Nucleotidzuckern bzw. - zuckerabkömmlingen eingesetzt wird.

4. Verfahren nach Anspruch 3, bei dem als Nucleosiddiphosphat UDP, ADP, TDP oder CDP und als Disaccharid Saccharose vorgesehen werden.

5. Verfahren nach Anspruch 3,
**dadurch gekennzeichnet,**
**daß** Saccharose-Synthase zusammen mit Epimerasen (a) und/oder Transferasen (b) zur Bildung eines aktivierten Zuckers oder Zuckerabkömmlings und
(a) Abwandlung ihres Zuckeranteils; oder
(b) Übertragung des Zuckeranteils auf andere Akzeptormoleküle eingesetzt wird.

6. Verfahren nach Anspruch 5 (b),
**dadurch gekennzeichnet,**
**daß** ein Zucker als Akzeptor vorgesehen wird.

7. Verfahren nach Anspruch 1 oder 2,
**dadurch gekennzeichnet,**
**daß** die so gewonnene Saccharose-Synthase zur Umsetzung von aktivierter Glucose bzw. Abkömmlingen derselben mit einem hydroxylgruppen-haltigen Akzeptor insbesondere einem Zucker zur Bildung von Glucosiden bzw. Abkömmlingen eingesetzt wird.

## Claims

1. Process for production of saccharose synthase **characterised in that** a saccharose synthase-containing crude extract is subjected to a sequence of purification steps which includes the application of an ultrafiltered extract of pH 8 containing 50 mM KCl onto a sepharose Q column and a gradient elution from the column at pH 8 with 50-500 mM KCl.

2. Process according to Claim 1, **characterised in that** a crude extract from swelled and mechanically disintegrated rice grains is subjected to the following purification steps:
- precipitation of accompanying proteins with PEG or aqueous 2-phase separation;
- application of the supernatant adjusted to pH 8 or of the correspondingly adjusted phase containing the enzyme activity onto a sepharose Q column;
- gradient elution at pH 8 with 50-500 mM KCl; and
- gel filtration column chromatography.

3. Process according to Claim 1 or 2, **characterised in that** the saccharose synthase thus obtained is used for the reaction of nucleoside diphosphates with di, tri or oligosaccharides or derivatives thereof with the formation of nucleotide-sugars or -sugar derivatives.

4. Process according to Claim 3, wherein UDP, ADP, TDP or CDP are provided as nucleoside diphosphate and saccharose as disaccharide.

5. Process according to Claim 3, **characterised in that** saccharose synthase together with epimerases (a) and/or transferases (b) is used for the formation of an activated sugar or sugar derivative and
(a) modification of its sugar portion; or
(b) transfer of the sugar portion onto other acceptor molecules.

6. Process according to Claim 5(b), **characterised in that** a sugar is provided as acceptor.

7. Process according to Claim 1 or 2, **characterised in that** the saccharose synthase thus obtained is used for the reaction of activated glucose or derivatives thereof with a hydroxyl group-containing acceptor in particular a sugar for the formation of glucosides or derivatives.

## Revendications

1. Procédé pour la fabrication de saccharose-synthase, **caractérisé par le fait qu'**un extrait brut contenant de la saccharose-synthase est soumis à une série d'étapes de purification, qui comprend le dépôt d'un extrait comportant du KCl 50 mM ultrafiltré de pH 8 sur une colonne de sépharose Q et une élution graduée sur la colonne à pH 8 avec du KCl 50 à 500 mM.

2. Procédé selon la revendication 1, **caractérisé par le fait qu'**un extrait brut issu de grains de riz gonflés et désagrégés mécaniquement est soumis aux étapes de purification suivantes :
- précipitation des protéines associées avec du polyéthylène glycol ou par une séparation aqueuse en deux phases ;
- dépôt du surnageant obtenu à pH 8 ou de la phase obtenue de manière correspondante comprenant l'activité enzymatique sur une colonne de sépharose Q ;
- élution graduée à pH 8 avec du KCl 50 à 500 mM ; et
- chromatographie en colonne par filtration sur gel.

3. Procédé selon la revendication 1 ou 2, **caractérisé par le fait que** la saccharose-synthase ainsi obtenue est utilisée pour la transformation de nucléosides diphosphates avec des disaccharides, des trisaccharides ou des oligosaccharides ou avec des dérivés de ceux-ci dans la formation de sucres de nucléotides ou de dérivés de sucres.

4. Procédé selon la revendication 3, dans lequel sont utilisés comme nucléosides diphosphates l'UDP, l'ADP, le TDP ou le CDP et comme disaccharide le saccharose.

5. Procédé selon la revendication 3, **caractérisé par le fait que** la saccharose-synthase est utilisée simultanément à des épimérases (a) et/ou des transférases (b) pour la formation d'un sucre activé ou d'un dérivé de sucre activé et
(a) la modification de sa fraction sucre ; ou
(b) le transfert de sa fraction sucre sur une autre molécule faisant fonction d'accepteur.

6. Procédé selon la revendication 5 (b), **caractérisé par le fait qu'**un sucre est prévu en tant qu'accepteur.

7. Procédé selon la revendication 1 ou 2, **caractérisé par le fait que** la saccharose-synthase ainsi obtenue est utilisée pour la transformation de glucose activé ou de dérivés activés de celui-ci avec un accepteur comportant un groupement hydroxyle, en particulier un sucre, pour la formation de glucosides ou de dérivés.
